# EUROPÄISCHE PATENTSCHRIFT

(11) **EP 2 080 532 B1**
(45) Veröffentlichungstag und Bekanntmachung des Hinweises auf die Patenterteilung: **01.12.2010**
(21) Anmeldenummer: 08000811.3
(22) Anmeldetag: 17.01.2008
(51) Int. Cl.: A61M 5/20, A61M 5/24, A61M 5/315

(54) **Kartusche für ein Autoinjektor und System bestehend aus einer solchen Kartusche und einem Autoinjektor**
Cartridge for an autoinjector and system made up of such a cartridge and an autoinjector
Cartouche pour un auto-injecteur et système constitué d'une telle cartouche et d'un auto-injecteur

(43) Veröffentlichungstag der Anmeldung: 22.07.2009
(73) Patentinhaber: Peter Loos und Arnold Neuhold Gewerbliche Schutzrechte GbR, 82418 Murnau (DE)
(72) Erfinder: Neuhold, Arnold, 82286 Huglfing (DE)
(74) Vertreter: HOFFMANN EITLE

(56) Entgegenhaltungen:
- WO-A-03/047663
- WO-A-2004/098687

## Beschreibung

### TECHNISCHES GEBIET

Die vorliegende Erfindung betrifft eine Kartusche bzw. Patrone für einen Autoinjektor sowie ein System aus Kartusche und Autoinjektor.

Unter einem Autoinjektor ist dabei ein medizinisches Instrument zu verstehen, welches zur Verabreichung einer Einzeldosis (Injektion) eines vorwiegend flüssigen Medikaments dient und das überwiegend zum Einsatz durch den Patienten selbst entwickelt wurde.

Die vorliegende Erfindung betrifft dabei eine Kartusche für einen Mehrwegautoinjektor, d. h. einen Autoinjektor, der wieder verwendet wird, wohingegen die Kartusche ein Einwegprodukt ist, das jeweils zum Verabreichen einer Einzeldosis dient. Nach dem Gebrauch muss die Kartusche folglich getauscht werden. Dabei muss der Autoinjektor vor dem Einsetzen einer neuen Kartusche und nach Entfernen der verbrauchten Kartusche gespannt werden.

Bei vorbekannten Systemen kann der Autoinjektor zu diesem Zweck zweiteilig gestaltet sein. Er wird zum Spannen getrennt und das abgetrennte Teil wird injektionsseitig in den Autoinjektor eingeführt, um diesen zu spannen.

Ein anderes System ist beispielsweise aus der WO 2004/098687 bekannt. Der dort beschriebene Autoinjektor wird zum Spannen und Entfernen einer in diesen Injektor eingelegten Spritze aufgeklappt. Dabei wird der Injektor gespannt. Im Anschluss wird eine neue Spritze eingelegt und der Injektor wieder zusammengeklappt. Der Autoinjektor ist dann für einen neuen Injektionsvorgang bereit.

### BESCHREIBUNG DER ERFINDUNG

Angesichts dieser vorbekannten Systeme bestand die Aufgabe der vorliegenden Erfindung darin eine Kartusche bzw. Patrone für einen Autoinjektor zu schaffen, die es ermöglicht den Autoinjektor beim Einsetzen einer neuen Kartusche in den Autoinjektor automatisch zu spannen, ohne dass zusätzliche Vorgänge notwendig wären. Gleichermaßen besteht die Aufgabe der vorliegenden Erfindung auch darin ein System aus einer solchen Kartusche und einem entsprechenden Autoinjektor zu schaffen.

Diese Aufgaben werden durch eine Kartusche mit den Merkmalen des Anspruchs 1 sowie ein System mit den Merkmalen des Anspruchs 11 gelöst.

Der vorliegenden Erfindung liegt der Gedanke zu Grunde die Kartusche derart zu gestalten, dass sie auf ihrer der Injektionsnadel abgewandten Seite beim Einsetzen mit einer Kolbenstange des Autoinjektors in Eingriff kommen kann, um diesen zu spannen, ohne dass die Kolbenstange den Kolben eines Fluidbehälters der Kartusche verschiebt, jedoch der Kolben zur Injektion durch die Kolbenstange verschiebbar ist.

Demgemäß umfasst die Kartusche für einen Autoinjektor einen Fluidbehälter mit einer Injektionsnadel an einem Ende und einem in dem Fluidbehälter verschiebbaren Kolben. Der Fluidbehälter ist insbesondere zur Aufnahme eines flüssigen Medikaments geeignet, kann aber auch ausgestaltet sein, um beispielsweise ein viskoses Medium, z. B. ein Gel aufzunehmen. Darüber hinaus ist der Fluidbehälter vorzugsweise ähnlich herkömmlicher Spritzen ohne Kolbenstange ausgestaltet, d. h. er kann länglich, hohlzylindrisch und aus Glas sein. Des Weiteren umfasst die Kartusche eine den Fluidbehälter aufnehmende Hülse. D. h. die Hülse kann gleichfalls länglich und hohlzylindrisch ausgestaltet sein und umgibt den Fluidbehälter wenigstens teilweise, um diesen aufzunehmen. Am injektionsnadelseitigen Ende ist die Hülse offen, wobei die Injektionsnadel und auch ein Teil des Fluidbehälters durch diese Öffnung vorragen können, aber nicht müssen. Am gegenüberliegenden Ende ist eine Spanneinrichtung vorgesehen, die dem Spannen des Autoinjektors dient. Diese Spanneinrichtung ist erfindungsgemäß zwischen einer ersten und einer zweiten Position bewegbar. In der ersten Position ist ein Zugang zum Kolben, zu dessen Betätigung versperrt, d. h. ein Verschieben des Kolbens durch den Autoinjektor beim Einsetzen der Kartusche in den Autoinjektor wird durch die Spanneinrichtung verhindert. In der zweiten Position, in die die Spanneinrichtung zu einem beliebigen Zeitpunkt nach dem Spannen, z. B. beim Auslösen des Injektionsvorgangs bewegbar ist, wird der Zugang zum Kolben gestattet, so dass ein Verschieben des Kolbens im Fluidbehälter und ein Injizieren des enthaltenen Mediums möglich ist. Mit anderen Worten wird beim Einsetzen der Kartusche die Spanneinrichtung beispielsweise mit der Kolbenstange des Autoinjektors in Eingriff gebracht und drückt diesen zum Spannen entgegen der Einstech- und Injektionsrichtung. Nach dem Wegbewegen der Spanneinrichtung gelangt die Kolbenstange mit dem Kolben in Eingriff und kann unmittelbar oder zeitverzögert diesen in Injektion-/Einstechrichtung verschieben.

Darüber hinaus ist es bevorzugt, dass die Kartusche der vorliegenden Erfindung zusammen mit dem Autoinjektor sowohl ein automatisches Einstechen der Injektionsnadel in die Haut als auch ein automatisches Ausdrücken des Fluidbehälterinhalts ermöglicht. Dabei ist es bevorzugt, dass der Fluidbehälter in Einstechrichtung im Autoinjektor verschiebbar ist. Dies wird erfindungsgemäß z. B. dadurch gelöst, dass der Fluidbehälter in Einstechrichtung in der Hülse (translatorisch) verschiebbar geführt ist, d. h. im Zusammenspiel mit der Kolbenstange des Autoinjektors und dem Kolben zunächst ein Verschieben des gesamten Fluidbehälters mit Kolben in Einstechrichtung erfolgt, ohne dass Medium ausgegeben wird.

Um ein Verschieben des Fluidbehälters in der Hülse bei entnommener Kartusche und vor dem Gebrauch zu verhindern, ist die Spanneinrichtung gemäß einer Ausführungsform derart gestaltet, dass sie den Fluidbehälter in der ersten Position in Einstechrichtung fixiert, so dass keine Verschiebung des Fluidbehälters in der Hülse möglich ist. In der zweiten Position, die gleichzeitig ein Zusammenwirken zwischen Kolben und Kolbenstange des Autoinjektors ermöglicht, wird die Verschiebung des Fluidbehälters dann gestattet, um den Fluidbehälter wie erwähnt translatorisch zu verschieben und dadurch ein Einstechen der Injektionsnadel in die Haut eines Verwenders zu stechen. Um den Weg der translatorischen Verschiebung zu begrenzen, kann in der Hülse ein Anschlag, z. B. eine Schulter, vorgesehen sein und der Fluidbehälter weist einen Gegenanschlag (z. B. einen Kragen) auf, die am Ende der Verschiebung in Eingriff kommen.

Aus herstellungstechnischen sowie Kostengründen ist es bevorzugt den Fluidbehälter, wie bereits oben erwähnt, basierend auf einer herkömmlichen Spritze auszugestalten, so dass ggf. sogar eine solche verwendbar ist. Es ist bekannt, dass herkömmliche Spritzen an ihren der Injektionsnadel abgewandten Ende einen Kragen aufweisen, der ggf. umlaufend sein kann. Gemäß einer besonderen Ausführungsform der vorliegenden Erfindung weist der Fluidbehälter demnach einen solchen Kragen auf und die Spanneinrichtung steht in der ersten Position mit dem Kragen in Eingriff, um den Fluidbehälter in der Hülse in Einstechrichtung zu fixieren. Z. B. kann in der Spanneinrichtung eine Nut entsprechend der Außenkontur des Kragens vorgesehen sein, in die der Kragen bei in erster Position befindlicher Spanneinrichtung ragt. Dieser Kragen kann gleichzeitig den oben erwähnten Gegenanschlag bilden.

Des Weiteren ist es auch herstellungstechnischen Gründen bevorzugt die Spanneinrichtung einstückig mit der Hülse auszugestalten. Dies kann vorzugsweise dadurch erzielt werden, dass die Spanneinrichtung als Schwenkarm ausgestaltet ist, d. h. die Spanneinrichtung ist über ein Filmscharnier mit dem verbleibenden Hülsenkörper verbunden und zwischen der ersten und zweiten Position verschwenkbar (rotatorische Bewegung). Alternativ ist es jedoch auch denkbar die Spanneinrichtung translatorisch bewegbar zu gestalten oder kombinierte Bewegungen (rotatorisch und translatorisch) zu verwirklichen.

Wird die Kartusche nach dem Gebrauch aus dem Autoinjektor entnommen, soll bevorzugterweise die Nadel unzugänglich in der Kartusche aufgenommen sein. Um dies zu ermöglichen schlägt die vorliegende Erfindung eine Kartusche vor, die zusätzlich ein Gehäuse umfasst, das relativ zu der Hülse verschiebbar ist. Z. B. kann, wenn die Hülse im Autoinjektor fixiert ist, ein Verschieben des Gehäuses relativ zur Hülse in Einstechrichtung erfolgen, um das Gehäuse über die Nadelspitze zu ziehen und die Nadel damit zu bedecken.

Zu diesem Zweck ist es bevorzugt die Hülse bei eingesetzter Kartusche lösbar im Autoinjektor zu fixieren. Dazu weist das Gehäuse eine Öffnung und die Hülse eine Fixiereinrichtung auf, die durch die Öffnung über eine Außenfläche des Gehäuses vorragt. Dabei kann zur Führung der Kartusche z. B. eine Nut in die Außenkontur des Gehäuses eingelassen sein, die sich in Einstechrichtung bzw. parallel dazu erstreckt und die Verriegelungseinrichtung kann über den Nutgrund als Außenfläche des Gehäuses vorragen.

Um gleichzeitig das Verschieben des Gehäuses relativ zur Hülse aber auch das Lösen der Fixiereinrichtung zum Entnehmen der Kartusche aus dem Autoinjektor zu ermöglichen, ist es bevorzugt, dass die Öffnung im Gehäuse länglich ausgestaltet ist, so dass die Verriegelungseinrichtung entlang der Längsrichtung der Öffnung, die in Einstechrichtung verläuft, verschiebbar ist. Darüber hinaus ist die Verriegelungseinrichtung als federnder Rasthaken ausgestaltet, der durch eine injektionsnadelseitige Anschlagfläche und eine sich von der Seite der Injektionsnadel weg erstreckende Rampe gebildet ist, die mit der Hülse verbunden ist, d. h. die Verbindung der Verriegelungseinrichtung mit der Hülse verwirklicht. Durch die Ausgestaltung dieses Rasthakens wird bei ausreichendem Verschieben des Gehäuses in Einstechrichtung die Rampe unter das der Injektionsnadel abgewandte Ende der länglichen Öffnung gezogen und drückt dadurch den Rasthaken mit seiner Anschlagfläche radial nach innen, so dass dieser außer Eingriff mit einem entsprechenden Verriegelungsgegenstück des Autoinjektors gelangen kann, so dass die Kartusche durch bloßes Ziehen am Gehäuse entnehmbar ist. Andererseits kann die Länge der Öffnung auch derart gestaltet sein, dass das Gehäuse eine gewisse Strecke verschoben werden kann, ohne dass der Rasthaken außer Eingriff mit dem Verriegelungsgegenstück gelangt, insbesondere um den erwähnten Nadelschutz zu verwirklichen.

Wie bereits zuvor erwähnt, ist gemäß einer bevorzugten Ausführungsform der Erfindung das Gehäuse relative zur Hülse verschiebbar, um einen Nadelschutz bereitzustellen. Dieser soll auch nach dem Gebrauch und dem Entnehmen der Kartusche bestehen, d. h. die Nadel soll auch nach dem Entnehmen der Kartusche nicht frei liegen. Damit das Gehäuse in dieser verschobenen Position, d. h. die Nadel vollständig umgebenen Position sicher gehalten wird, ist es bevorzugt, dass das Gehäuse eine Aussparung umfasst und die Hülse mit einer Rastnase versehen ist, die durch Verschieben der Hülse relativ zu dem Gehäuse entgegen der Einstechrichtung bzw. des Gehäuses relativ zur Hülse in Einstechrichtung mit der Öffnung in Eingriff bringbar ist und ansonsten lösbar mit der Aussparung in Eingriff steht und dann ein Verschieben des Gehäuses relativ zur Hülse in Einstechrichtung verhindert.

Darüber hinaus ist das Gehäuse gemäß einer bevorzugten Ausführungsform an seinem injektionsnadelseitigen Ende mit einer Öffnung versehen, um ein Durchtreten der Injektionsnadel beim Einstechvorgang zu gestatten. Zum Entnehmen der Kartusche sowie um das Gehäuse weiter über die Nadelspitze zu ziehen, weist das Gehäuse ferner eine Einrichtung zum Greifen des Gehäuses auf, die in Form eines umlaufenden Kragens ausgestaltet sein kann.

Ferner ist es bevorzugt, das Gehäuse mit einer Rastnase zu versehen, die vorzugsweise an dem der Injektionsnadel abgewandten Ende vorgesehen ist und in eine Aussparung der Spanneinrichtung greift. Dabei kann die Aussparung derart gestaltet sein, dass sich die Rastnase in Einstechrichtung eine gewisse Strecke bewegen kann, um ein Verschieben des Gehäuses relativ zur Hülse auch in einem Zustand zu gestatten, in dem sich die Spanneinrichtung in der ersten Position befindet. Dies ist jedoch nicht zwingend notwendig. Durch die Anordnung der Aussparung in der Spanneinrichtung kommt die Rastnase außer Eingriff mit der Aussparung, wenn die Spanneinrichtung in die zweite Position bewegt wird, d. h. die Aussparung wird gleichfalls mitbewegt und dadurch der Eingriff zwischen der Rastnase des Gehäuses und der Aussparung der Spanneinrichtung gelöst, so dass bei in der zweiten Position befindlicher Spanneinrichtung das Gehäuse relativ zur Hülse frei beweglich ist, wobei die zusätzlichen Rasthaken und Rastnasen der Hülse und die Öffnung und Aussparung der Gehäuse zu berücksichtigen sind.

Zum Betätigen des Autoinjektors weist der Autoinjektor eines Systems gemäß der vorliegenden Erfindung eine Betätigungseinrichtung auf, die vorzugsweise manuell betätigbar ist und die auf eine Betätigung die Spanneinrichtung aus der ersten in die zweite Position bewegt.

Darüber hinaus ist es bevorzugt die Betätigungseinrichtung als Schwenkhebel auszugestalten, da ein solcher leicht herstellbar und gut zu bedienen ist. Zum Bewegen der Spanneinrichtung ist der Schwenkhebel an seinem der Schwenkachse abgewandten Ende zur Zusammenwirkung mit der Spanneinrichtung ausgestaltet.

Insbesondere, wenn die Spanneinrichtung ein Schwenkarm ist, ist es bevorzugt, dass die Schwenkachse des Schwenkhebels des Autoinjektors parallel zur Schwenkachse des Schwenkarms verläuft, wobei die beiden der Schwenkachse abgewandten Ende des Schwenkhebels und des Schwenkarms bei Betätigung der Betätigungseinrichtung direkt oder indirekt in Anlage kommen, um den Schwenkarm aus der ersten in die zweite Position zu verschwenken.

Damit beim Spannen des Autoinjektors, während dem der Autoinjektor möglicherweise voll in der Hand liegend gehalten wird und auch Druck auf die Betätigungseinrichtung ausgeübt wird, kein sofortiges Wiederauslösen erfolgt, ist es möglich, das der Schwenkachse des Schwenkhebels abgewandte Ende des Schwenkhebels mit einem Auslöseabschnitt zu versehen, der um eine zu der Schwenkachse des Schwenkhebels parallele Ausweichschwenkachse verschwenkbar ist. Dies ermöglicht es beim Einsetzen einer neuen Kartusche, dass die sich in der ersten Position befindliche Spanneinrichtung gegen den Auslöseabschnitt drückt und sich dieser um seine Angleichschwenkachse verschwenkt, d. h. ausweicht. In dieser Position verbleibt der Auslöseabschnitt auf der Oberfläche der Spanneinrichtung liegend ohne diese aus der ersten in die zweite Position bewegen zu können. D. h. ein Auslösen des Autoinjektors ist in dieser Position nicht möglich. Das "scharf" machen des Autoinjektors erfolgt durch Verschwenken der Betätigungseinrichtung und ein Verschiebeelement (siehe später).

Wie bereits erwähnt weist der Autoinjektor ein Verriegelungsgegenstück auf, das im Bereich seines einstechseitigen Endes ausgebildet und mit dem ein Teil der Anschlagfläche des Rasthakens der Hülse in Eingriff bringbar ist, um die Hülse relativ zum Autoinjektor bzw. dessen Gehäuse lösbar festzulegen, wohingegen das Gehäuse der Kartusche in Einstechrichtung verschieblich bleibt. Das Lösen erfolgt durch Bewegen des Gehäuses in Einstechrichtung, so dass die Rampe des Rasthakens unter das Gehäuse läuft und den Rasthaken außer Eingriff mit dem Verriegelungsgegenstück drückt, so dass die Kartusche entfernt werden kann (siehe oben).

Um das Gehäuse stets die Injektionsnadelspitze verdeckend anzuordnen, wenn der Autoinjektor nicht auf der Haut aufgesetzt ist, umfasst der Autoinjektor gemäß einer bevorzugten Ausführungsform ferner ein in Einstechrichtung beaufschlagtes Verschiebeelement, das mit dem Gehäuse derart zusammenwirkt, dass bei in den Autoinjektor eingesetzte Kartusche das Gehäuse relativ zur Hülse in Einstechrichtung gedrückt wird, d. h. über die Injektionsnadel, um deren Spitze zu verdecken.

Dieses Verschiebeelement wird gemäß einer bevorzugten Ausführungsform gleichzeitig dazu genutzt den Auslöseabschnitt in Einstechrichtung zu beaufschlagen. Allerdings kann der Auslöseabschnitt auch um seine Schwenkachse in Einstechrichtung federbeaufschlagt sein, d. h. selber in diese Richtung federn. Durch beide Möglichkeiten kann durch Ziehen am Betätigungselement und damit Bewegen des Auslöseabschnitts von der Spanneinrichtung weg der Autoinjektor "scharf" gemacht werden, wobei der Auslöseabschnitt wieder mit der Spanneinrichtung zusammenwirken kann, um letztere aus der ersten in die zweite Position zu bewegen.

Weitere Merkmale, Vorteile und Kombinationen der vorliegenden Erfindung werden aus der folgenden Beschreibung bevorzugter Ausführungsformen ersichtlich, die unter Bezugnahme auf die begleitenden Zeichnungen erfolgt.

In den Zeichnungen zeigen:
Fig. 1 eine Kartusche gemäß der vorliegenden Erfindung a) perspektivisch, b) in einem Längsschnitt entlang der Linie B-B in Fig. 1c, c) in einer ersten Seitenansicht, d) in einen zweiten Seitenansicht, die relativ zur ersten Seitenansicht um 90° gedreht ist und e) in einem Längsschnitt entlang der Linie A-A in Fig. 1d;
Fig. 2 einen Autoinjektor mit einer eingesetzten Kartusche gemäß Fig. 1 in a) einer Seitenansicht und b) einem Schnitt entlang der Linie A-A in Fig. 2a;
Fig. 3 den Autoinjektor aus Fig. 2 in einem Längsschnitt entlang der Linie A-A in Fig. 2 jedoch um 90° verdreht;
Fig. 4 einen Längsschnitt durch die Kartusche nach Abziehen einer Schutzkappe gleich dem Schnitt in Fig. 1e;
Fig. 5 den Autoinjektor aus Fig. 2 im Schnitt entlang der Linie A-A aus Fig. 2a im ausgelösten Zustand;
Fig. 6 einen Längsabschnitt durch die Kartusche im ausgelösten Zustand gleich dem Schnitt in Fig. 1e;
Fig. 7 einen Längsabschnitt durch die Kartusche nach dem Entfernen aus dem Autoinjektor gleich dem Schnitt in Fig. 1b.

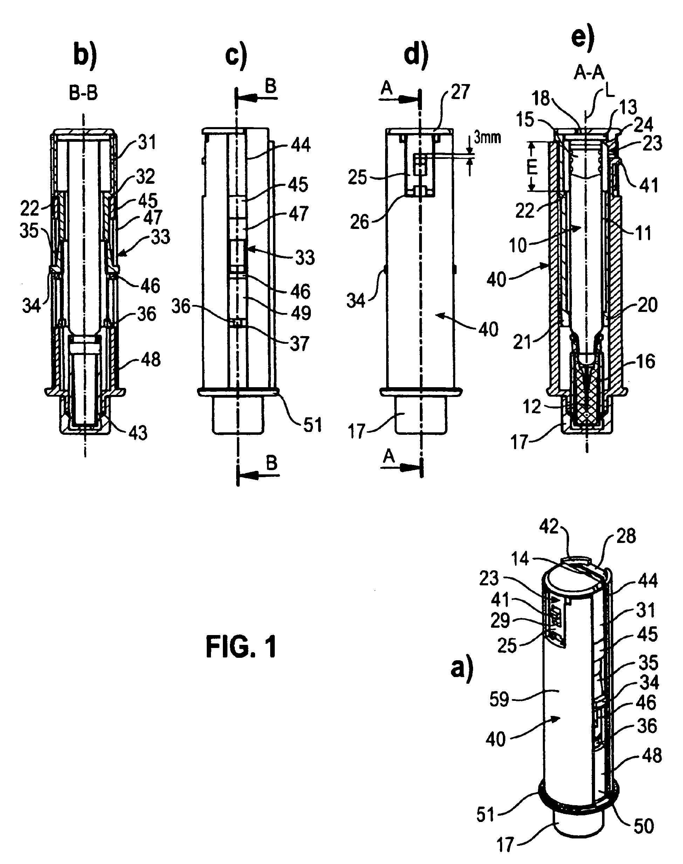

Im Folgenden wird die Erfindung anhand einer bevorzugten Ausführungsform beschrieben, wobei zunächst auf die in Fig. 1 dargestellte Kartusche eingegangen wird.

Die Kartusche umfasst einen Fluidbehälter 10, der ähnlich einer herkömmlichen Spritze aus Glas oder einem glasähnlichen Werkstoff gebildet ist. Der Fluidbehälter 10 weist einen das Fluid aufnehmende Aufnahmeabschnitt 11 auf, an dessem einen Ende (Vorderende) eine Injektionsnadel vorgesehen (angebracht oder integral ausgestaltet) ist. An dem der Injektionsnadel abgewandten Ende des Aufnahmeabschnitts 11 ist ein Kragen 13 ausgebildet. Der Aufnahmeabschnitt 11 ist hohlzylinderförmig und mit einem kreisrunden Innenabschnitt ausgebildet. Der Kragen erstreckt sich über die Außenkontur des Aufnahmeabschnitts 11 umlaufend hinaus, ist jedoch nicht kreisrund ausgestaltet, sondern hat zwei gegenüberliegende abgeflachte Kanten 14 (siehe Fig. 1a). In dem Aufnahmeabschnitt 11 ist vor der Injektion vorzugsweise ein flüssiges Medikament enthalten. Darüber hinaus ist in dem Aufnahmeabschnitt 11 in Längsrichtung L verschieblich ein Kolben 15 geführt. Dieser Kolben 15 weist keine Kolbenstange auf und dient dazu durch ein Verschieben in Richtung der Injektionsnadel 12, das in dem Aufnahmeabschnitt 11 enthaltene flüssige Medikament durch die Injektionsnadel 12 auszugeben. Die Injektionsnadel ist im Anlieferzustand, d. h. ungebrauchten Zustand der Kartusche mit einer Schutzkappe 16 bedeckt. Mit dieser Schutzkappe kann ein Abzieher 17 verbunden sein, der bei in dem Injektor eingesetzter Kartusche von außen zugänglich ist und durch einen Anwender greifbar, um zum Gebrauch die Schutzkappe 16 abzuziehen.

Des Weiteren umfasst die dargestellte Kartusche eine Hülse 20, die an ihrem der Injektionsnadel 12 zugewandten Ende 21 eine Öffnung aufweist, durch die ein Teil des Aufnahmeabschnitts 11 sowie die Injektionsnadel 12 ragt und deren Innendurchmesser an den Außendurchmesser des Aufnahmeabschnitts 11 angepasst ist, um den Fluidbehälter 10 in Längsrichtung L in der Hülse 20 verschieblich zu lagern bzw. zu führen.

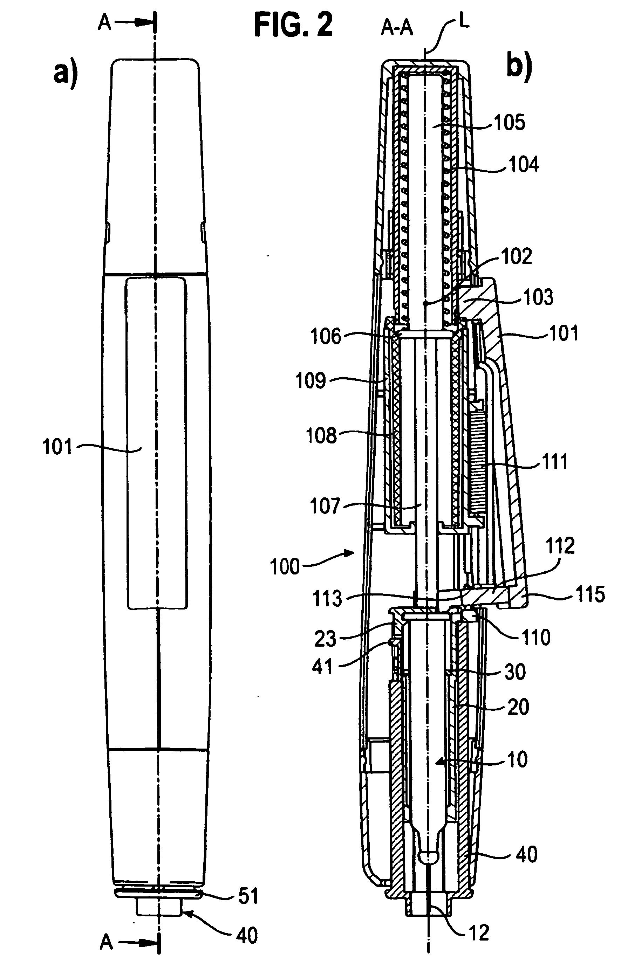

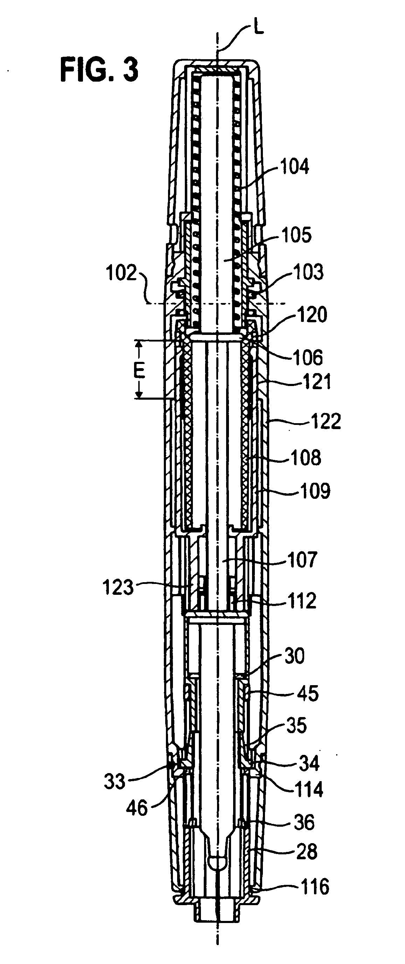

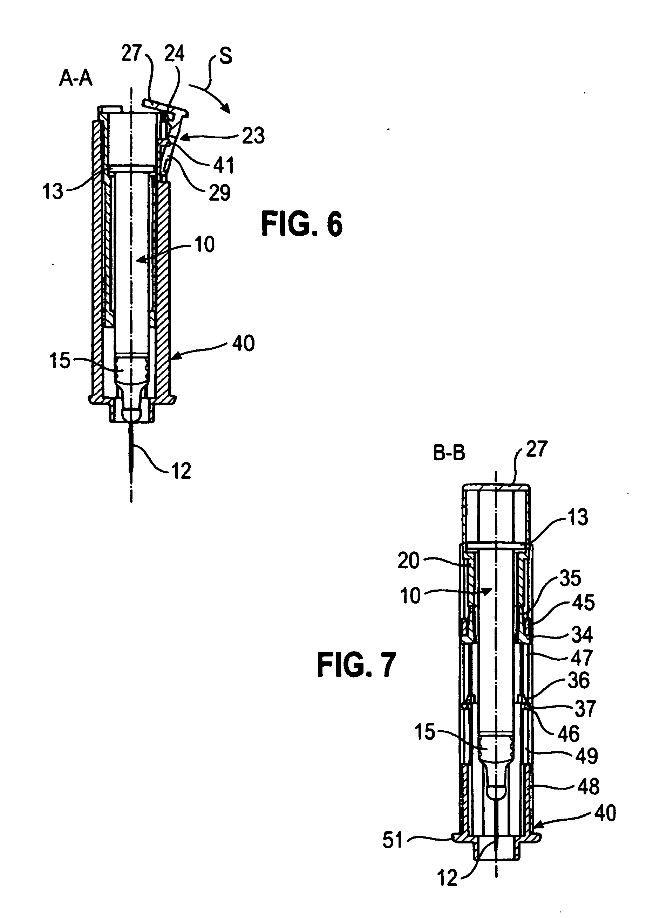

Darüber hinaus ist die Hülse 20 in einem Abstand E von der Unterkante des Kragens 13 mit einer Schulter 22 ausgestaltet. Der Abstand E ist derart ausgestaltet, dass der Fluidbehälter 10 während des Injektionsvorgangs, d. h. beim Auslösen des Autoinjektors, in Längsrichtung L soweit verschiebbar ist (Abstand E), dass die Injektionsnadel in die Haut eines Anwenders einstechbar ist. Mit anderen Worten beschränkt die Schulter 22 während dieser Phase des Auslösens den Weg der Längsverschiebung des Fluidbehälters 10 in der Hülse 20. Auf der Schulter 22 kann ein separates Dämpfungselement 30 (siehe Fig. 2b und 3) vorgesehen sein, das beim Einstechvorgang den Schlag des Kragens 13 auf die Schulter 22 dämpft, um (Glas-)Bruch zu vermeiden. Dieses Dämpfungselement 30 kann auch integral mit der Hülse 20 ausgebildet sein, z. B. in Form von federnd vorragenden Stegen oder ähnlichem. Ferner ist die Hülse 20 an ihrem der Injektionsnadel 12 abgewandten Ende mit einer Spanneinrichtung 23 ausgestattet, die bei der vorliegenden Ausführungsform als Schwenkhebel ausgestaltet ist und derart mit dem Rest (Körper) der Hülse 20 verbunden ist, dass ein Verschwenken um eine nicht dargestellte Schwenkachse in Richtung des Pfeils S (siehe Fig. 6) möglich ist.

Die Spanneinrichtung 23 ist gebildet durch einen sich im Wesentlichen in Längsrichtung L erstreckenden Schwenkhebel (Schwenkarm) 25, der über ein Filmscharnier 26 mit dem Rest der Hülse 20 (dem Hülsenkörper) verbunden ist, um die Schwenkbewegung zu ermöglichen. An dem der Injektionsnadel 12 abgewandten Ende des Schwenkhebels 25 erstreckt sich im Wesentlichen senkrecht dazu eine Art Deckelabschnitt, der eine der Injektionsnadel 12 abgewandte Öffnung 18 im Fluidbehälter 10 zumindest soweit bedeckt, dass die später beschriebene Kolbenstange 105, 106, 107 nicht mit dem Kolben 15 im Fluidbehälter 10 in Eingriff kommen kann, so dass dieser beim Einsetzen der Kartusche in den Autoinjektor nicht verschoben wird.

Des Weiteren wird zwischen dem deckelartigen Abschnitt 27 und einer zusätzlich in Richtung der Injektionsnadel von dem Deckelabschnitt bzw. dessen Unterkante beabstandeten radial nach innen ragenden Nase 24 eine Art Nut gebildet, in die der Kragen 13 des Fluidbehälters 10 ragt, so dass im Zustand der in Fig. 1 dargestellt ist, der Fluidbehälter 10 durch die Nase 24 in Längsrichtung L fixiert wird, d. h. in diesem Zustand nicht relativ zur Hülse 20 verschoben werden kann.

Darüber hinaus ist der verbleibende Teil der Hülse im Bereich des Kragens (in Fig. 1 mit 28 bezeichnet) an die Außenkontur des Kragens 13 angepasst, so dass ein Verdrehen des Fluidbehälters 10 in der Hülse 20 nicht möglich ist.

Der Schwenkhebel 25 der Spanneinrichtung 23 weist eine Aussparung 29 auf, in die eine später beschriebene Rastnase 41 des Gehäuses 40 ragt, wobei die Aussparung 29 rechteckig gestaltet ist und mit einer Größe bzw. Länge oder Dimension, dass ein Verschieben der Rastnase 41 in Längsrichtung L über einen gewissen Hub (z. B. 3 mm) nach oben und/oder unten (z. B. ca. 6 mm) gestattet ist.

Die Kartusche umfasst ferner ein Gehäuse 40, das die Hülse 20 und den Fluidbehälter 10 aufnimmt. Wie bereits erwähnt umfasst das Gehäuse 40 eine Rastnase 41. Diese Rastnase 41 ragt, wie bereits erwähnt, in die Aussparung 29 im Schwenkhebel 25 der Spanneinrichtung 23 und ist in dieser in Längsrichtung einen gewissen Hub in beide Richtungen entlang der Längsrichtung verschiebbar.

Des Weiteren weist das Gehäuse an seinem der Injektionsnadel abgewandten Ende eine Öffnung 42 auf, deren Innendurchmesser so gestaltet ist, dass die Hülse 20 eingeschoben werden kann. An dem injektionsnadelseitigen Ende ist eine weitere Öffnung 43 vorgesehen, die kleiner gestaltet ist und durch die die Injektionsnadel 12 beim Injektionsvorgang geschoben wird. Diese Öffnung 43 kann beispielsweise kreisrund ausgestaltet sein.

Im Bereich der Öffnung 42 ist eine von der Öffnung 42 ausgehende rechteckige Nut bzw. Aussparung 44 an diametral gegenüberliegenden Seiten des Gehäuses 40 ausgebildet. Die Hülse 20 umfasst einen zugehörigen Vorsprung 31, der eine Schulter 32 bildet, die an der in Längsrichtung L injektionsnadelseitigen Begrenzung der Aussparung 44 anliegt, um eine Relativverschiebung der Hülse 20 zum Gehäuse 40 in Längsrichtung L zu begrenzen.

Dabei wird die injektionsnadelseitige Begrenzung der Aussparung 44 durch einen Steg 45 gebildet. Es versteht sich, dass der Steg und auch die nachfolgend nur für eine Seite beschriebenen Elemente ein diametral gegenüberliegendes Gegenstück aufweisen, das gleich ausgestaltet ist (siehe Fig. 1b). Der Steg zusammen mit einem weiteren in Längsrichtung L vom Steg 45 beabstandeten Steg 46 definieren eine Öffnung 47 in dem Gehäuse 40. Die Hülse 20 umfasst eine Verriegelungseinrichtung 33. Diese wird gebildet durch eine injektionsnadelseitige Anschlagfläche 34 sowie eine sich davon entgegen der Einstechrichtung erstreckende Rampe 35. Dabei verjüngt sich die Rampe 35 von der Anschlagfläche 34 ausgehend entgegen der Einstechrichtung. Die Fixiereinrichtung 33 ist durch die Anwendung des oberen, d. h. der Injektionsnadel abgewandten Endes der Rampe mit dem Rest der Hülse federnd verbunden. Die Dimensionierung der Fixiereinrichtung 33 und der Öffnung 47 ist derart, dass eine Verschiebung der Fixiereinrichtung 33 relativ zur Öffnung 47 bzw. dem Gehäuse 40 möglich ist. Dabei ist die Öffnung 47 rechteckig gestaltet und auch die Fixiereinrichtung 33 hat in ihrer Draufsicht (Fig. 1c) eine rechteckige Form. In dem in Fig. 1 dargestellten Zustand der Kartusche liegt die Anschlagfläche 34 an der der Injektionsnadel abgewandten Seite des Stegs 46 an.

Des Weiteren ist zwischen dem Steg 46 und dem injektionsnadelseitigen Ende 48 des Gehäuses 40 eine weitere in der Draufsicht rechteckige Öffnung 49 gebildet. In diese ragt eine Rastnase 36 der Hülse 20, die eine der Injektionsnadel zugewandte Anschlagsfläche 37 aufweist, die im Zustand in Fig. 1 mit dem der Injektionsnadel abgewandten Ende des Abschnitts 48 der Hülse 40 zusammenwirkt. Auch die Rastnase 36 ist so ausgestaltet, dass sie in der Öffnung 49 in Längsrichtung L relativ zum Gehäuse 40 verschiebbar ist.

Wie es am besten aus Fig. 1a ersichtlich ist, sind die Stege 45, 46 so ausgestaltet, dass ihre Außenfläche relativ zur verbleibenden Außenfläche 59 des Gehäuses 40 nach innen (radial nach innen) versetzt ist. Darüber hinaus ist auch in dem der Injektionsnadel zugewandten Abschnitt 48 des Gehäuses 40 eine Nut 50 ausgestaltet, die mit den Stegen 45, 46 fluchtet. Der Vorsprung 31, welcher die Schulter 32 bildet, die auf dem Steg 45 aufliegt und der zur Hülse 20 gehört, ist gleichfalls mit einem solchen Außendurchmesser bzw. einer solchen Außenkontur gestaltet, dass die Oberfläche des Vorsprungs 31 mit der Oberfläche des Stegs 45 und damit mit dem Steg 46 sowie der Nut bzw. dem Nutgrund der Nut 50 fluchtet. Die Anschlagfläche 34 der Fixiereinrichtung 33 ragt jedoch über diese fluchtend gebildete Oberfläche um einen gewissen Abstand vor, um die Kartusche im Autoinjektor zu fixieren (siehe später).

Schließlich ist im Unterabschnitt des Gehäuses 40, d. h. dem Abschnitt der der Injektionsnadel zugewandt ist, ein umlaufender Greifkragen 51 ausgebildet, der wie später erläutert werden wird, dazu dient es einem Anwender zu ermöglichen die Kartusche wieder aus dem Autoinjektor zu entfernen.

Im Folgenden wird unter Bezugnahme auf die Fig. 2 und 3 ein Autoinjektor beschrieben, in den eine Kartusche, wie sie in Bezug auf Fig. 1 erläutert wurde, einsetzbar ist. Diese ist gleichfalls in den Figuren dargestellt.

Der Autoinjektor weist ein Gehäuse 100 auf, das mehrteilig ausgestaltet sein kann. Mit dem Gehäuse verbunden ist eine Betätigungseinrichtung 101 zum Auslösen des Autoinjektors, d. h. zum Einstechen der Injektionsnadel 12 in die Haut eines Verwenders und Injizieren des flüssigen Medikaments im Fluidbehälter 10 durch die Injektionsnadel 12. Die Betätigungseinrichtung 101 ist als Schwenkhebel ausgestaltet und um eine Schwenkachse 102 verschwenkbar. Um die Betätigungseinrichtung 101 im Gehäuse 100 um die Schwenkachse 102 verschwenkbar zu lagern, ist die Betätigungseinrichtung 101 an ihrem Hinterende (der Injektionsnadel abgewandt) gabelförmig 103 ausgestaltet und umgreift einen Betätigungskolben.

Der Betätigungskolben setzt sich aus drei Abschnitten zusammen, einem Abschnitt 105, einem Abschnitt 107 und einem Abschnitt 106 zwischen den Abschnitten 105 und 107. Alle Abschnitte sind vorzugsweise im Querschnitt kreisrund. Dabei ist der Abschnitt 107 in seinem Außendurchmesser derart ausgelegt, dass er beim Auslösen in die Öffnung 18 des Fluidbehälters 10 eintreten kann, um den Kolben 15 zu verschieben. Der Abschnitt 105 ist im Durchmesser im Querschnitt größer ausgestaltet jedoch kleiner als der Durchmesser des kragenförmigen Abschnitts 106. Dadurch ist es möglich ein Beaufschlagungselement in Form der Schraubenfeder 104 zwischen einem oberen (der Injektionsnadel abgewandt) Ende des Gehäuses 100 und der Schulter 106 abzustützen, so dass beim Auslösen die Feder 104 den Kolben 105, 106, 107 in Einstechrichtung in Längsrichtung L bewegen kann.

Darüber hinaus ist eine Dämpfungshülse 108 aus einem elastischen Material vorgesehen, wobei der Kragen 106 bzw. sein Außenumfang mit der Dämpfungshülse 108 in einem flexiblen Formeingriff steht, der im Verlaufe der Vorschubbewegung des Injizierkolbens bestehen bleibt, sich jedoch örtlich verändert und zur Dämpfung einer Formänderungskraft wirksam wird, um einen gleichmäßigen Injektionsvorgang stets mit der gleichen Kraft zu bewerkstelligen. Hinsichtlich dieser Ausgestaltung wird der Fachmann auf die WO 2007/020090 der gleichen Anmelderin verwiesen, die dieses Prinzip im Detail erläutert. Die Dämpfungshülse 108 ist in einem in dem Gehäuse 100 des Autoinjektors in Längsrichtung L verschiebbaren Aufnahmeelement 109 gelagert.

Des Weiteren weist der Autoinjektor ein Vorschub- oder Verschiebeelement 110 auf, das ebenfalls in Längsrichtung L verschieblich angeordnet ist. Zwischen dem Vorschubelement 110 und dem Aufnahmeelement 109 ist eine Zugfeder 111 angeordnet, welche das Vorschubelement 110 in Einschubrichtung beaufschlagt.

Das der Injektionsnadel zugewandte Ende (untere Ende) der Betätigungseinrichtung 101 weist einen gabelförmigen Abschnitt 112 auf, der sich im Wesentlichen senkrecht zur Längserstreckung der Betätigungseinrichtung 101 von diesem weg erstreckt. Dieser gabelförmige Bereich 112 (Gabel 112) ist ausgestaltet, um den Abschnitt 107 des Kolbens umgreifen zu können, so dass ein Verschwenken der Betätigungseinrichtung 101 um die Schwenkachse 102 möglich ist. Die in Fig. 2b linker Hand dargestellte Stirnseite 113 des gabelförmigen Elements 112, die der Betätigung der Spanneinrichtung 23 dient, kommt mit der in Fig. 2b rechter Hand dargestellten Stirnseite der Spanneinrichtung 23 in Anlage.

Wie es am besten aus Fig. 3 ersichtlich ist, weist der Autoinjektor 100 ferner zwei diametral gegenüberliegende Verriegelungsgegenstücke 114 auf, die in der durch die Oberflächen der Vorsprünge 31, der Stege 45 und 46 sowie der Nuten 48 gebildeten Nut der Kartusche laufen, wenn diese in den Autoinjektor eingesetzt wird. Gleichermaßen sind am offenen unteren Ende des Autoinjektors Führungen 115 vorgesehen, die beim Einführen der Kartusche gleichfalls in die Nut greifen, um die Kartusche gegen ein Verdrehen zu sichern und beim Einsetzen bzw. -schieben zu führen.

Im Folgenden wird die Funktionsweise des beschriebenen Systems aus Kartusche und Autoinjektor unter Bezugnahme auf die Fig. 1-7 beschrieben.

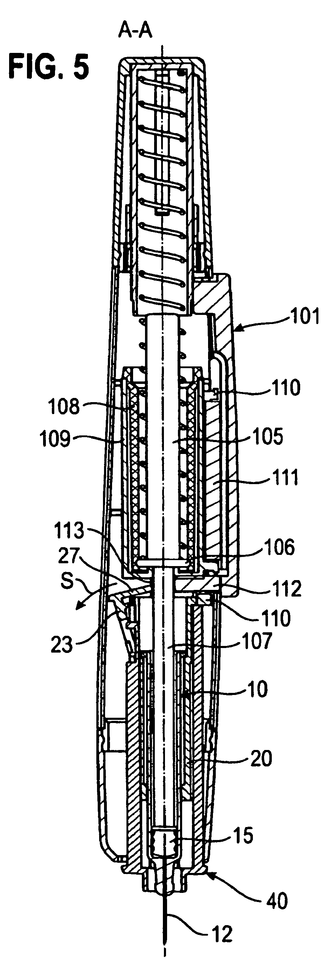

Vor der Verwendung des Autoinjektors, d. h. vor dem Verabreichen einer Injektion liegt der Autoinjektor in einer ungespannten Form vor, d. h. der Kolben 105, 106, 107 befindet sich in der in Fig. 5 dargestellten Stellung. Jedoch ist (entgegen der Darstellung in Fig. 5) keine Kartusche in den Autoinjektor eingesetzt.

In diesem Zustand nimmt der Anwender eine Kartusche, wie sie in Fig. 1 dargestellt ist und richtet die durch unter anderem den Vorsprung 31 und die Aussparung 44 gebildete Nut zu den Führungsvorsprüngen 115 aus. Dann schiebt der Anwender die Kartusche entgegen der Einstechrichtung, d. h. in den Fig. 2, 3 und 5 nach oben in dem Autoinjektor. Dabei laufen die Fixiereinrichtungen 33 (die federnd ausgestaltet sind) an den Verriegelungsgegenstücken 114 vorbei (sie werden radial nach innen gedrückt) und die Fixiereinrichtungen 33 federn zurück in ihre Ausgangsstellung (radial nach außen). Dabei kommen die in Richtung der Injektionsnadel gerichteten Anschlagsflächen 34 in Eingriff mit den nach oben gewandten Seiten der Verriegelungsgegenstücke 114, so dass die Hülse 20 in Längsrichtung L in Einstechrichtung (nach unten) fixiert ist. Das Gehäuse 40 hingegen bleibt nach wie vor, begrenzt unter anderem durch den Eingriff der Rastnase 41 mit der Aussparung 29, in der Hülse längs verschieblich. Dadurch, dass die Hülse 20 in Längsrichtung L fixiert ist, ist auch der Fluidbehälter 10 in Längsrichtung fixiert. Der Kragen 13 wird durch die Rastnase 24 der Hülse 20 gehalten. Durch das Einführen der Kartusche wird der Kolben nach oben verschoben, so dass auch die Aufnahme 109 und die elastische Dämpfungshülse 108 mit nach oben verschoben werden bis sie gegen den gabelförmigen Abschnitt 103 des Betätigungselements laufen, um das Betätigungselement in die in Fig. 2b dargestellte Ausgangsstellung zurückzuverschwenken bzw. in diese Stellung zu beaufschlagen, in der es dann durch das Vorschubelement 110 (siehe oben) gesperrt ist. In dieser Stellung liegt der Abschnitt 107 des Kolben mit seiner Stirnseite (der Injektionsnadel zugewandt) auf der Oberseite des Deckelabschnitts 27 der Spanneinrichtung 23 auf. Die Hülse 20 ist damit zwischen dem Kolbenabschnitt 107 und den Verriegelungsgegenstücken verklemmt und unbeweglich.

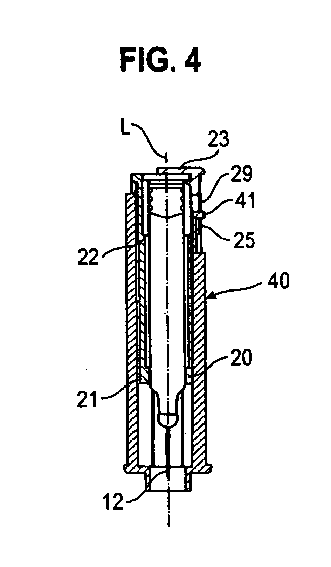

In diesem Zustand zieht der Anwender an dem Abzieher 17, wodurch die Schutzkappe 16 von der Injektionsnadel 12 bzw. dem Fluidbehälter 10 gelöst wird und die Injektionsnadel 12 "frei liegt". Beim Entfernen des Abziehers 17 und der Schutzkappe 16 wird das Gehäuse 40 durch den Reibungseingriff mit nach unten gezogen und gleichzeitig durch die Beaufschlagung durch das Vorschubelement 110, das mit dem oberen Ende des Gehäuses 40 in Anlage steht durch die Zugfeder 111 in Einstechrichtung beaufschlagt, so dass sich das Gehäuse 40 relativ zu der im Autoinjektor in Längsrichtung fixierten Hülse 20 und dem Fluidbehälter 10 in Einstechrichtung verschiebt und somit die Spitze der Injektionsnadel 16 vollständig umgibt (siehe Zustand der Kartusche in Fig. 4). Durch diese Bewegung, die in einem Bereich bzw. einer Größenordnung von 3 mm liegt, wird die Betätigungseinrichtung 101 gesperrt. Durch diese Gehäusebewegung bewegt sich auch das Vorschubelement 110 3 mm nach unten, d. h. in Richtung der Injektionsnadel. Dadurch greift ein Element des Vorschubelements 110 derart in das Betätigungselement 101 ein, dass dieses nicht gedrückt bzw. betätigt werden kann. Die Vorrichtung ist gesperrt.

Das Sperren der Betätigungseinrichtung 101 kann jedoch auch anders ausgestaltet sein. So könnte z. B. der gabelförmige Abschnitt 112 des Betätigungselements 101 um die in Fig. 2b angedeutete Ausgleichschwenkachse 116 verschenkbar gelagert sein (andere Ausführungsform) und zwar relativ zu dem verbleibenden Abschnitt des Betätigungselements 101, der sich im Wesentlichen parallel zur Längsachse L erstreckt. Wird eine neue Kartusche eingeführt, befindet sich der Betätigungshebel 101 in der eingedrückten, d. h. verschwenkten Position (nicht dargestellt). Wird die Kartusche eingeführt kommt damit der gabelförmige Abschnitt 112 mit der Oberseite des Deckelabschnitts 27 in Eingriff und wird im Uhrzeigersinn um die Ausgleichschwenkachse 116 nach oben verschwenkt. Bei dieser Ausführungsform ist das Vorschubelement 110 derart ausgestaltet, dass es nicht nur die Hülse 20 in Einstechrichtung beaufschlagt, sondern auch den gabelförmigen Abschnitt 112 der Betätigungseinrichtung 101. Ist die Kartusche eingeführt und soll der Injektionsvorgang ausgelöst werden, so verschwenkt ein Anwender das Betätigungselement 101 aus dem Gehäuse 100 des Autoinjektors, d. h. entgegen des Uhrzeigersinns um die Schwenkachse 102 (dies wird ggf. durch die Beaufschlagung des gabelförmigen Elements durch die elastische Dämpfungshülse 108 unterstützt). Dabei bewegt sich das gabelförmige Element 112 auf der Oberfläche des Deckelabschnitts 27 in Fig. 2 z. B. nach rechts. Erreicht es das rechter Hand dargestellte Ende des Deckelabschnitts 28 wird es durch das Vorschubelement 110 nach unten gedrückt und gelangt in die in Fig. 2b dargestellte Stellung. Der Autoinjektor ist in dieser Stellung scharf.

Bei der ursprünglich beschriebenen Ausführungsform, bei der das Vorschubelement 110 eine Betätigung in der Stellung in Fig. 2b blockiert, wird ein Auslösen dadurch gewährleistet, dass der Autoinjektor 100 auf die Haut aufgesetzt wird. Beim Aufbringen einer gewissen Kraft auf den Autoinjektor in Längsrichtung (in Einstechrichtung) wird das Gehäuse 40 entgegen der Federkraft der Zugfeder 111, d. h. entgegen der Kraft, die durch das Vorschubelement 110 auf die Hülse aufgebracht wird entgegen der Einstechrichtung verschoben und hebt die Sperre auf. Dann ist ein Auslösen bzw. Verschwenken der Betätigungseinrichtung 101 möglich.

Wird das Betätigungselement 101 in der scharfen Stellung (Fig. 2b) im Uhrzeigersinn um die Schwenkachse 102 verschwenkt, so drückt das gabelförmige Element 112 mit seiner Stirnseite 113 gegen die Stirnseite des Deckelabschnitts 27 der Spanneinrichtung 23, so dass diese um das Filmscharnier 26 entgegen dem Uhrzeigersinn verschwenkt wird.

Die Schwenkbewegung, die in Fig. 6 im Uhrzeigersinn folgt, ist durch den Pfeil S auch in Fig. 5 entgegen dem Uhrzeigersinn dargestellt. Durch das Wegschwenken der Spanneinrichtung 23 kommt einerseits der Kragen 13 außer Eingriff mit der durch die Rastnase 24 und die Unterseite des Deckelabschnitts 27 gebildete Nut und andererseits gelangt die Stirnseite des Kolbenabschnitts 107 mit der oberen Stirnseite des Kolbens 15 in Eingriff. Der Fluidbehälter 10 ist nun in der Hülse 20 frei in Längsrichtung verschieblich, wobei der Reibungswiderstand zwischen der Innenseite des Aufnahmeabschnitts 11 und der Außenfläche des Kolbens 15 größer ausgestaltet sein kann als der Reibungswiderstand zwischen der Außenfläche des Fluidbehälters 10 insbesondere des Aufnahmeabschnitts 11 und der Hülse 20 bzw. deren Führung 21. Das Aufnahmeelement 109 für die Dämpfungshülse 108 bewegt sich zusammen mit der Dämpfungshülse 108 und dem Kolben 105, 106, 107 in Einstechrichtung nach vorne. Dies wird dadurch erzielt, dass das Aufnahmeelement 109 mit federnden Nasen 120 ausgestaltet ist, die im Bereich 121 des Gehäuses 101 des Autoinjektors an dessen Innenfläche anliegen, so dass ein radiales Ausweichen dieser federnden Nasen 120 nach außen verhindert ist. Somit wird der Kragen 106 des Kolbens über die Dämpfungshülse 108 und die Nasen 120 gegriffen, so dass der Aufnahmeabschnitt 109 zusammen mit dem Kolben 105, 106, 107 in Einstechrichtung verschoben wird. Diese mechanische Kopplung ist in der oben erwähnten WO 2007/0200090 genauer erläutert. Darüber hinaus weist der Aufnahmeabschnitt 109 Einstechelemente 123 auf, die in dem in Fig. 3 dargestellten unausgelösten Zustand mit dem Deckelabschnitt der Spanneinrichtung 23 in Kontakt stehen. Bei in die zweite Position bewegter Spanneinrichtung 23 sind diese Einstechelemente 123 derart ausgestaltet, dass sie mit der in Fig. 3 nach oben gerichteten Außenfläche des Kragens 13 des Fluidbehälters 10 in Eingriff gelangen. Durch die Vorschubbewegung des Aufnahmeelements 109 werden auch die Einstechelemente 123 nach vorne bewegt und diese Vorschubbewegung auf den Kragen 13 übertragen, so dass der Fluidbehälter 10 in Einstechrichtung bewegt wird. Dadurch wird der Fluidbehälter 10 in Längsrichtung L um den Betrag E nach vorne geschoben bis der Kragen 13 auf der Schulter 22 bzw. dem Dämpfungselement 30 anschlägt und zum liegen kommt, wodurch die Längsbewegung begrenzt wird. Dieser Vorschub bewirkt ein Einstechen der Injektionsnadel 12 in die Haut eines Anwenders. Dieser Zustand ist für die Kartusche alleine in Fig. 6 dargestellt. Der Greifkragen 51 bildet einen Anschlag, damit er stets von außen greifbar bleibt.

Nachdem der Einstechvorgang erfolgt ist und der Fluidbehälter 10 nicht mehr weiter in Längsrichtung L in Einstechrichtung vorgeschoben werden kann, drückt der Kolbenstangenabschnitt 107 den Kolben 50 in Injektionsrichtung Einstechrichtung nach vorne (siehe Fig. 5), um den Inhalt des Aufnahmeabschnitts vorzugsweise das flüssige Medikament durch die Injektionsnadel zu injizieren, d. h. auszugeben. Dabei gleiten die federnden Nase 120 des Aufnahmeelements 109 entlang der Innenfläche 121 des Gehäuses, wobei diese Innenfläche mit einem kleineren Innendurchmesser etwa die Länge E aufweist. D. h. ist der Einstechvorgang abgeschlossen, so vergrößert sich der Innendurchmesser des Gehäuses 101 im Bereich 122, so dass die federnden Nasen 120 radial nach außen ausweichen können. Zu diesem Zeitpunkt wird der Kragen 106 in der Dämpfungshülse 108 freigegeben und der Kolben 105, 106, 107 vorgeschoben, um den Fluidbehälterinhalt zu injizieren. Dabei wandert der Kragen 106 unter Dämpfungswirkung der Dämpfungshülse 108 entlang der Innenfläche der Dämpfungshülse 108. Dann ist der Injektionsvorgang abgeschlossen.

Hebt der Anwender den Autoinjektor wieder von der Haut ab, drückt das Vorschubelement 110 das Gehäuse 40 wieder in Einstechrichtung nach vorne, so dass gleichzeitig mit dem Abheben auch die Injektionsnadel 12 wieder bedeckt wird und nicht frei liegt.

Durch das Wegschwenken der Spanneinrichtung ist das Gehäuse relative zu der Hülse 20 in Längsrichtung frei verschieblich. Zum Entfernen der Kartusche aus dem Autoinjektor greift ein Anwender den Greifkragen 51 und zieht an dem Gehäuse 40. Dadurch bewegt sich das Gehäuse 40 relativ zur Hülse 20 nach unten. Dabei gelangt der Steg 45 in Eingriff mit der Rampe 35. Dabei wandert der Steg 45 vom entgegen der Injektionsnadel gerichteten Ende der Rampe in Richtung der Anschlagfläche 34. Dadurch werden die Fixiereinrichtungen 33 radial nach innen gedrückt, so dass der Eingriff zwischen den Fixiereinrichtungen 33 und den Verriegelungsgegenstücken 114 des Autoinjektors gelöst wird und die Kartusche in Längsrichtung entnommen werden kann. Zum Zeitpunkt, zu dem der Eingriff gelöst wird, sind auch die Rastnasen 36 am Steg 46 vorbeigerutscht und die in Richtung der Injektionsnadel gerichtete Anschlagsflächen 37 der Rastnasen 36 stehen mit den durch die Stege 46 gebildeten Begrenzungen der Öffnungen 47 in Eingriff, so dass eine Bewegung der Hülse 20 relativ zum Gehäuse 40 in Einstechrichtung nicht mehr möglich ist (siehe Fig. 7), so dass die Injektionsnadel 12 zuverlässig durch das Gehäuse 40 verdeckt und geschützt bleibt. Damit werden Verletzungen nach Entnahme der Kartusche vermieden.

Im Anschluss wird die Kartusche entsorgt oder einem Recyclingvorgang zugeführt.

Danach wiederholt sich der oben beschriebene Vorgang erneut.

Es versteht sich, dass die obige Beschreibung nicht auf die beschriebene Ausführungsform beschränkt ist. Vielmehr sind verschiedene Modifikationen und Änderungen innerhalb des Umfangs der Erfindung, wie er durch die folgenden Patentansprüche definiert ist, möglich.

## Patentansprüche

1. Kartusche für einen Autoinjektor, umfassend
einen Fluidbehälter (10) mit einer Injektionsnadel (12) an einem Ende und einem in dem Fluidbehälter verschiebbaren Kolben (15), **dadurch gekennzeichnet, dass**:
eine den Fluidbehälter (10) aufnehmende Hülse (20), die am injektionsnadelseitigen Ende offen ist und am gegenüberliegenden Ende eine Spanneinrichtung (23) umfasst, die zwischen einer, einen Zugang zum Kolben (15) zu dessen Betätigung versperrenden, ersten Position und einer, den Zugang zum Kolben (15) gestattenden, zweiten Position bewegbar ist.

2. Kartusche nach Anspruch 1, bei der der Fluidbehälter (10) in Einstechrichtung in der Hülse (20) verschiebbar geführt ist und die Spanneinrichtung (23) derart gestaltet ist, dass sie den Fluidbehälter (10) in der ersten Position in Einstechrichtung fixiert und in der zweiten Position die Verschiebung des Fluidbehälters (10) gestattet.

3. Kartusche nach Anspruch 2, bei der der Fluidbehälter (10) an seinem der Injektionsnadel abgewandten Ende einen Kragen (13) aufweist und die Spanneinrichtung (23) in der ersten Position mit dem Kragen in Eingriff steht.

4. Kartusche nach einem der vorstehenden Ansprüche, bei der die Spanneinrichtung (23) als Schwenkarm (25) ausgestaltet ist, der zwischen der ersten und der zweiten Position verschwenkbar ist.

5. Kartusche nach einem der vorstehenden Ansprüche, ferner umfassend ein Gehäuse (40) in dem die Hülse (20) entlang der Einstechrichtung verschiebbar aufgenommen ist.

6. Kartusche nach Anspruch 5, wobei das Gehäuse (40) eine Öffnung (47) und die Hülse (20) eine Fixiereinrichtung (33) aufweist, die lösbar mit der Öffnung (47) in Eingriff steht.

7. Kartusche nach Anspruch 6, bei der die Fixiereinrichtung über eine Außenfläche des Gehäuses vorragt.

8. Kartusche nach Anspruch 6 oder 7, bei der die Öffnung (47) länglich ist und die Fixiereinrichtung ein federnder Rasthaken ist, der durch eine injektionsnadelseitige Anschlagsfläche (34) und eine sich von der Seite der Injektionsnadel weg erstreckende Rampe (25), die mit der Hülse (20) verbunden ist, gebildet ist.

9. Kartusche nach einem der Ansprüche 6 bis 8, bei der das Gehäuse (40) eine Aussparung (49) aufweist und die Hülse (20) mit einer Rastnase (36) versehen ist die durch Verschieben des Gehäuses (40) relativ zur Hülse (20) entgegen der Einstechrichtung lösbar mit der Aussparung (49) in Eingriff steht und beim Verschieben mit der Öffnung (47) in Eingriff bringbar ist.

10. Kartusche nach einem der Ansprüche 5 bis 9, bei der das Gehäuse (40) eine Rastnase (41) und die Spanneinrichtung (23) eine Aussparung (29) aufweist, in die die Rastnase des Gehäuses greift.

11. System umfassend einen Autoinjektor und eine Kartusche nach einem der vorstehenden Ansprüche.

12. System nach Anspruch 11, bei dem der Autoinjektor eine Betätigungseinrichtung (101) umfasst, die auf eine Betätigung die Spanneinrichtung (23) aus der ersten in die zweite Position bewegt, wobei die Betätigungseinrichtung (101) bevorzugterweise ein Schwenkhebel ist, der zum Bewegen der Spanneinrichtung an seinem der Schwenkachse (102) abgewandten Ende mit der Spanneinrichtung (23) zusammenwirkt.

13. System nach Anspruch 12, bei dem die Kartusche die Merkmale des Anspruchs 4 umfasst und die Schwenkachse (102) des Schwenkhebels parallel zur Schwenkachse des Schwenkarms (25) verläuft, wobei das seiner Schwenkachse abgewandte Ende des Schwenkhebels bei Betätigung der Betätigungseinrichtung mit dem seiner Schwenkachse abgewandten Ende des Schwenkarms in Anlage kommt, um den Schwenkarm aus der ersten in die zweite Position zu verschwenken.

14. System nach Anspruch 12 oder 13, bei dem das seiner Schwenkachse (102) abgewandte Ende des Schwenkhebels mit einem Auslöseabschnitt (112) versehen ist, der um eine zu der Schwenkachse des Schwenkhebels parallele Ausweichschwenkachse (115) verschwenkbar ist.

15. System nach einem der Ansprüche 11 bis 14, bei dem die Kartusche die Merkmale des Anspruchs 8 umfasst und der Autoinjektor im Bereich seines einstechseitigen Endes ein Verrieglungsgegenstück (114) aufweist mit dem ein Teil der Anschlagfläche (34) des Rasthakens der Hülse (20) in Engriff bringbar ist, um die Kartusche lösbar im Autoinjektor zu fixieren.

16. System nach einem der Ansprüche 11 bis 14, bei dem die Kartusche die Merkmale des Anspruchs 5 umfasst und der Autoinjektor ein in Einstechrichtung beaufschlagtes Verschiebelement (110) aufweist, das mit dem Gehäuse (40) zusammenzuwirkt, um bei in den Autoinjektor eingesetzter Kartusche das Gehäuse (40) relativ zur Hülse in Einstechrichtung zu beaufschlagen

17. System nach Anspruch 16 und 14, bei dem das Verschiebelelement (110) mit dem Auslöseabschnitt (112) zusammenwirkt, um diesen in Einstechrichtung zu beaufschlagen.

## Claims

1. Cartridge for an auto-injector comprising a fluid container (10) with an injection needle (12) on one end and a piston (15) which can be displaced in the fluid container, **characterised in that** a sleeve (20) which receives the fluid container (10) and which is open at the end on the injection-needle side and at the opposite end comprises a clamping device (23) which can be moved between a first position blocking access to the piston (15) for actuation thereof and a second position permitting access to the piston (15).

2. Cartridge according to claim 1, in which the fluid container (10) is guided to be displaceable in the sleeve (20) in insertion direction and the clamping device (23) is designed such that it fixes the fluid container (10) in the first position in insertion direction and permits displacement of the fluid container (10) in the second position.

3. Cartridge according to claim 2, in which the fluid container (10) has a collar (13) at its end facing away from the injection needle and the clamping device (23) engages with the collar in the first position.

4. Cartridge according to one of the above claims, in which the clamping device (23) is designed as a pivoting arm (25) which can be pivoted between the first and the second position.

5. Cartridge according to one of the above claims, further comprising a housing (40) in which the sleeve (20) is received to be displaceable along the insertion direction.

6. Cartridge according to claim 5, wherein the housing (40) has an opening (47) and the sleeve (10) has a fixing device (33) which engages with the opening (47) to be releasable.

7. Cartridge according to claim 6, in which the fixing device projects beyond an outer surface of the housing.

8. Cartridge according to claim 6 or 7, in which the opening (47) is elongated and the fixing device is a resilient locking hook which is formed by a stop surface (34) on the injection-needle side and a ramp (25) extending away from the side of the injection needle and which is connected to the sleeve (10).

9. Cartridge according to one of claims 6 to 8, in which the housing (40) has a recess (49) and the sleeve (20) is provided with a locking nose (36) which engages with the recess (49) to be releasable by displacement of the housing (40) relative to the sleeve (20) counter to the insertion direction and can be engaged with the opening (47) during displacement.

10. Cartridge according to one of claims 5 to 9, in which the housing (40) has a locking nose (41) and the clamping device (23) has a recess (29), into which the locking nose of the housing engages.

11. System comprising an auto-injector and a cartridge according to one of the above claims.

12. System according to claim 11, in which the auto-injector comprises an actuating device (101) which on actuation moves the clamping device (23) from the first to the second position, wherein the actuating device (101) is preferably a pivoting lever which cooperates with the clamping device (23) to move the clamping device at its end facing away from the pivoting axis (102).

13. System according to claim 12, in which the cartridge comprises the features of claim 4 and the pivoting axis (102) of the pivoting lever runs parallel to the pivoting axis of the pivoting arm (25), wherein the end of the pivoting lever facing away from its pivoting axis comes to rest with the end of the pivoting arm facing away from its pivoting axis on actuation of the actuating device to pivot the pivoting arm from the first to the second position.

14. System according to claim 12 or 13, in which the end of the pivoting lever facing away from its pivoting axis (102) is provided with a release section (112) which can be pivoted about an alternative pivoting axis (115) which is parallel to the pivoting axis of the pivoting lever.

15. System according to one of claims 11 to 14, in which the cartridge comprises the features of claim 8 and the auto-injector has a locking counter-piece (114) in the region of its end on the insertion side, with which one part of the stop surface (34) of the locking hook of the sleeve (20) can be engaged to fix the cartridge to be releasable in the auto-injector.

16. System according to one of claims 11 to 14, in which the cartridge comprises the features of claim 5 and the auto-injector has a displacement element (110) which is acted upon in insertion direction and which cooperates with the housing (40) to act upon the housing (40) relative to the sleeve in insertion direction when the cartridge is inserted in the auto-injector.

17. System according to claim 16 and 14, in which the displacement element (110) cooperates with the release section (112) to act upon the latter in insertion direction.

## Revendications

1. Cartouche pour un auto-injecteur, comprenant
un récipient à fluide (10) avec une aiguille d'injection (12), à une extrémité, et un piston (15) déplaçable dans le récipient à fluide, **caractérisé en ce qu'**
une douille (20), recevant le récipient à fluide (10), ouverte à l'extrémité située côté aiguille d'injection et comprenant un dispositif de serrage (23) à l'extrémité opposée, est déplaçable entre une première position, bloquant un accès au piston (15) pour son actionnement, et une deuxième position, permettant l'accès au piston (15).

2. Cartouche selon la revendication 1, pour laquelle le récipient à fluide (10) est guidé de manière déplaçable dans la douille (20), dans le sens du piquage, et le dispositif de serrage (23) est configuré de manière qu'il fixe le récipient à fluide (10) dans la première position dans le sens du piquage et, dans la deuxième position, permette le déplacement du récipient à fluide (10).

3. Cartouche selon la revendication 2, pour laquelle le récipient à fluide (10) présente, sur son extrémité opposée à l'aiguille d'injection, une collerette (13), et, dans la première position, le dispositif de serrage (23) est mis en prise avec la collerette.

4. Cartouche selon l'une des revendications précédentes, pour laquelle le dispositif de serrage (23) est configuré sous forme de bras pivotant (25), susceptible de pivoter entre la première et la deuxième position.

5. Cartouche selon l'une des revendications précédentes, comprenant en outre un boîtier (40), dans lequel la douille (20) est logée de manière déplaçable dans la direction du piquage.

6. Cartouche selon la revendication 5, dans laquelle le boîtier (40) présente une ouverture (47) et la douille (20) présente un dispositif de fixation (33), mis en prise de manière désolidarisable avec l'ouverture (47).

7. Cartouche selon la revendication 6, pour laquelle le dispositif de fixation fait saillie sur une face extérieure du boîtier.

8. Cartouche selon la revendication 6 ou 7, pour laquelle l'ouverture (47) est oblongue et le dispositif de fixation est un crochet d'encliquetage élastique, formé par une face de butée (34) située côté aiguille d'injection, et une rampe (25), s'écartant du côté de l'aiguille d'injection, reliée à la douille (20).

9. Cartouche selon l'une quelconque des revendications 6 à 8, pour laquelle le boîtier (40) présente un évidement (49) et la douille (20) est munie d'un ergot d'encliquetage (36) mis en prise de manière désolidarisable avec l'évidement (49), par déplacement du boîtier (40) par rapport à la douille (20), à l'encontre du sens de piquage, et susceptible d'être mis en prise avec l'ouverture (47) lors du déplacement.

10. Cartouche selon l'une quelconque des revendications 5 à 9, pour laquelle le boîtier (40) présente un ergot d'encliquetage (41) et le dispositif de serrage (23) présente un évidement (29), dans lequel s'engage l'ergot d'encliquetage du boîtier.

11. Système comprenant un auto-injecteur et une cartouche selon l'une des revendications précédentes.

12. Système selon la revendication 11, pour lequel l'auto-injecteur comprend un dispositif d'actionnement (101) qui, sur un actionnement, fait passer le dispositif de serrage (23) de la première à la deuxième position, le dispositif d'actionnement (101) étant de préférence un levier pivotant, coopérant avec le dispositif de serrage (23) à son extrémité opposée à l'axe de pivotement (102) pour déplacer le dispositif de serrage.

13. Système selon la revendication 12, pour lequel la cartouche comprend les caractéristiques de la revendication 4 et l'axe de pivotement (102) du levier pivotant s'étendant parallèlement à l'axe de pivotement du bras pivotant (25), l'extrémité, opposée à son axe de pivotement, du levier pivotant, lors de l'actionnement du dispositif d'actionnement, venant en appui avec l'extrémité, opposée à son axe de pivotement, du bras pivotant, pour faire pivoter le bras pivotant, de la première à la deuxième position.

14. Système selon la revendication 12 ou 13, pour lequel l'extrémité, opposée à son axe de pivotement (102), du levier pivotant est munie d'un tronçon de déclenchement (112), susceptible de pivoter autour d'un axe d'échappement par pivotement (115) parallèle à l'axe de pivotement du levier pivotant.

15. Système selon l'une des revendications 11 à 14, pour lequel la cartouche comprend les caractéristiques de la revendication 8 et l'auto-injecteur présente, dans la zone de son extrémité située côté piquage, une pièce de verrouillage (114) avec laquelle une partie de la face de butée (34) du crochet d'encliquetage de la douille (20) est susceptible d'être mise en prise, pour fixer la cartouche, de manière désolidarisable, dans l'auto-injecteur.

16. Système selon l'une des revendications 11 à 14, pour lequel la cartouche comprend les caractéristiques de la revendication 5 et l'auto-injecteur présente un élément de déplacement (110) sollicité dans le sens du piquage, coopérant avec le boîtier (40), pour, lorsque la cartouche est insérée dans l'auto-injecteur, solliciter le boîtier par rapport à la douille, dans le sens du piquage.

17. Système selon les revendications 16 et 14, pour lequel l'élément de déplacement (110) coopère avec le tronçon de déclenchement (112) pour solliciter celui-ci dans le sens du piquage.
